# EUROPEAN PATENT SPECIFICATION

(11) **EP 2 367 955 B1**
(45) Date of publication and mention of the grant of the patent: **09.01.2013**
(21) Application number: 09799723.3
(22) Date of filing: 26.11.2009
(51) Int. Cl.: C12Q 1/68

(54) **OLIGONUCLEOTIDES FOR THE DETECTION OF ASPERGILLUS SPECIES**
OLIGONUKLEOTIDE ZUM NACHWEIS VON ASPERGILLUS-SPEZIES
OLIGONUCLEOTIDES POUR LA DETECTION D ESPECES ASPERGILLUS

(30) Priority: 26.11.2008 HU 0800722
(43) Date of publication of application: 28.09.2011
(73) Proprietor: University of Debrecen, 4010 Debrecen (HU)
(72) Inventor: DR. BÍRÓ, Sándor, H-4225 Debrecen (HU); DR. BIRKÓ, Zsuzsanna, H-4225 Debrecen (HU); PAHOLCSEK, Melinda, H-2737 Ceglédbercel (HU)
(74) Representative: Svingor, Adam
(86) International application number: PCT/IB2009/055369
(87) International publication number: WO 2010/061351

(56) References cited:
- US-A- 5 958 693
- FLORENT MARTINE ET AL: "Prospective evaluation of a polymerase chain reaction-ELISA targeted to Aspergillus fumigatus and Aspergillus flavus for the early diagnosis of invasive aspergillosis in patients with hematological malignancies" JOURNAL OF INFECTIOUS DISEASES, vol. 193, no. 5, March 2006 (2006-03), pages 741-747, XP002572583 ISSN: 0022-1899

## Description

### The field of the invention

The field of the invention generally is diagnostic microbiology, particularly the species specific detection and identification of Aspergillus species.

The invention relates to oligonucleotides that are specific for the fungi belonging to *Aspergillus* genus, and which are able to hybridize to *facC* gene homologues present in those fungi and show homology with the *facC* gene of *Streptomyces griseus* 45H, the sequence of said homologous gene is identical with any sequences of SEQ ID NO 118 to 120. These oligonucleotides make possible the detection and identification the members of the *Aspergillus* genus, specifically the *Aspergillus fumigatus* or *Aspergillus terreus.*

### Background of the invention

The saprophytic *Aspergillus* species are ubiquitous in our environment, however as opportunistic pathogens they only cause systemic diseases/infections in immunocompromised hosts/patients (those with AIDS, acute leukemia, those under intensive cytotoxic chemotherapies). Despite of this besides the *Candida* species *Aspergillus* species are the second most common causative agents of nosocomial fungal systemic infections with the incidence of 1/20 000. The explanation of this phenomenon can be found in the changes of the state of the art in the last quarter century, because artificial immunosuppressive treatments drastically increased the number of invasive mycoses.

Despite of the rapid development in antifungal therapy during the past decade, the aspergillosis cases remain a major cause of the infection-related morbidity and mortality in developed countries.

Besides the *Candida* species the mayor causative agents of the highly devastating systematic mycoses are mainly caused by the filamentous fungi of the *Aspergillus* genus, such as *Aspergillus fumigatus, A. terreus, A. flavus, A. niger* and A. *nidulans* (Pagano et al., 2006). Numerous articles confirm that other human pathogens like *Neosartorya* (Guarro et al., 2002) and *Chaetomium* (Anandi et al., 1989, Abbott et al., 1995; Yeghen et al., 1996) also play important role in the development of said disease.

*Aspergillus* species rarely cause disease in healthy persons and these infections cannot disperse among people either. Conidia enters the body by inhalation since the *Aspergillus* genus relating filamentous fungi are ubiquitous in our environment. They lead a saprophytic life in the soil. Besides infections caused by fungi spores originated from rotten organic residues (compost pitches) and *Aspergillus* species consumed with pepper, coffee or peanut, nosocomial infections are also important, e.g. infections arising during hospital treatment (especially distributed by the air conditioning apparatuses of the intensive car or other departments) (Vonberg, 2006).

Due to the limited or total immunocompromised state of the individuals these infections may become invasive and in spite of the fact that in the status of the primary disease a distinct improvement is showed, the secondary evolved infections may lead to death.

The infection may become systemic due to the immune defect. After a given time (in months or in years) the infection becomes systemic and through the blood system disseminates in the body e.g. into the central nervous system, liver or kidneys (Vidal *et al..,* 2005; Hummel *et al.,* 2006).

Depending on where the conidia are able to colonize we classify aspergillosis in three main categories. Aspergilloma or mycetoma, allergic bronchopulmonary aspergillosis or aspergilloma of the lungs and finally the invasive aspergillosis, which last one is deadly in almost 100% of the cases.

Survival depends on early started antifungal therapy.

The prevention would be of great significance in case of those patients that belong to the risk group. In their cases regular cost effective screening would be important. The prophylactic use of antimycotics may be able to decrease the frequency of the disease.

Furthermore the species level identification of Aspergilluses is also of great importance, since it is a prerequisite of the targeted antifungal therapy because different species response differently to a given antifungal treatment, not to mention that this way the spread of resistant fungi species may be controlled and decreased. For example *Aspergillus terreus* is known to be resistant to Amphotericin B, which is among the first line options in antifungal therapy and which lately is combined with different Echinocandins, like with Voriconazol (Segal *et al.,* 2006).

### Diagnosis of aspergillosis

The reliable diagnosis is hampered by some difficulties since the symptoms are not specific and the causative agents of mycosis are hard to identify due to the presence of other causative and concomitant microbes. Furthermore very important is the species level detection, which is the prerequisite of the targeted antifungal therapy.

The most reliable confirmation of the presence of fungus in the attacked tissues is only possible by analyzing or histologically examining fungi cultures originating from appropriate samples, however in most of the cases these procedures are mainly post mortem, on the other hand these cultivating tests are rarely appropriate for species level detection.

Microbiological and histopathological methods are time consuming and they often need samples from biopsies that are not always appropriate due to the risk associated with the disease, because in many patients the biopsy itself is risky.

Different imaging procedures like X-ray, CT, MRI examinations and the results of cultivations from the sputum, nose phlegm, BAL (broncoalveolar lavage) facilitate the diagnosis only in far-gone statement (White *et al.,* 2006a; Erjavec, Verweij, 2002).

Nowadays besides the imaging procedures and culture based confirmations commercially available *Aspergillus* diagnostic methods can be:
- **serological procedures/assays** like the latex agglutination and ELISA (Enzyme-Linked Immunosorbent Assay) or the sandwich-ELISA, which is the improved form of the previous. In case of these assays the antigen is the in the plasma circulating fungi cell wall component, the (1→5)-ß-galactofuranosil side chain of (1→3)-ß-D-glucan molecule or other termo stable polysaccharide component like the galactomannan, which consists of a 'non-immunogen', called "mannan core" central core, and contains immunoactive side chains (a ligand használható), like galactofuranosil units.
- **DNA based and the combination of these** (Florent *et al.,* 2006; Denning, 2006; Williamson *et al.,* 2000). The advantage of the latter hybrid method is that it is able to combine the high rate (94-100%) specificity of PCR reactions with the high rate (85-100%) sensitivity characteristic in serological methods (Aquino *et al.,* 2007).

The greatest advantage of the serological methods is the rapid applicability. Opposite to the culture based methods these can show results within 3 hours (Aquino *et al.,* 2007), however since these methods screen for ubiquitously present fungal cell wall components, they are not capable for species level detection.

Therefore it is needed to combine these methods with other DNA based detection methods (nested PCR, quantitative-real time-PCR). These are mainly based on the conserved sequences of ribosomal RNA genes (see e.g: EP 979312).

The DNA based methods are highly common because they are fast, easily reproducible and well applicable. Depending on the attributes of the target gene these are able to show high specificity (almost 100%) (Aquino *et al,* 2007).

Other factors such as the origin of the specimens (blood, BAL-fluid), the efficiency of the DNA isolation, the structure and specificity of the primers (are they able to build primer dimers or other non specific amplified fragments), the type of the PCR reaction (two step, nested, seminested, quantitative-real time-PCR) influences the sensitivity to a great extent.

If fungal fragments can be found in biological samples then they can be detected after the appropriate elaboration in less than one day. Recently the methods based on quantitative real time PCR (Q-RT-PCR) systems are the most widely used (Bolehovska *et al.,* 2006; White *et al.,* 2006/b). The Q-RT-PCR methods are capable of monitoring the amplification procedure cycle by cycle. The quantitative detection of the amplified fragments takes place by measuring fluorescence signals. Another great advantage of the method is that the assays are handled in separated and closed tubes which reduce the possibility of environmental contamination during the processing.

Opposite to the serological methods the appropriately performed assays can identify *Aspergilli* on species level (Erjavec, Verweij, 2002).

### False positive results

In the case of serological methods a great percentage (14%) of false positive results occur in the case of a patient group who were treated right before the diagnosis by the combination of beta lactam antibiotics and beta lactamase inhibitors like PIPERACILLIN-TAZOBACTAM, AMPICILLIN-SULBACTAM and AMOXICILLIN-CLAVULANIC ACID or with other antibiotics such as Penicillin-G, Ceftriaxon, Imipenem, Ciprofloxacin, Vancomocin, Gentamicin and so on (Aquino *et al.,* 2007).

These cases can be explained by artificial contamination. Artificial contamination can happen accidentally during sample preparation in the case of a healthy patient and in patients under antibiotics treatment artificial contaminants are introduced into the bloodstream, in the cell wall of beta lactam antibiotics producing *Acremonium* genus galactofuranosil ligands can be found as well (Florent *et al.,* 2006).

In case of real time PCR system processed reactions based on the detection of specific ribosomal RNA genes that are present generally in fungi it is also unavoidable even in case of healthy people that their investigated serum, sputum, saliva and from different body fluid (like BAL) contains different kind of fungi nucleic acids from the environment and damaged by the defense mechanism of the healthy immunsystem (Bolehovska *et al.,* 2006).

The drawbacks of the presently available methods for detecting aspergillus are clearly shown by the fact that these methods are not in clinical use (Cesaro et al., 2008, see e.g. page 2, column 1). A person skilled in the art is well aware of the drawbacks of the presently available methods (see e.g. White et al., Donelly et al., Lewis et al., 2006).

Regarding the previously mentioned facts it can be stated that according to the state of art taking a reliable und unambiguous diagnosis needs the combination of different diagnostic methods which require significant amount of time, energy and financial input. However the screening of the *Aspergillosis* cases should form a part of preventive routine diagnosis.

### The object of the invention

The object of this invention was to set up a diagnostic method that by itself supplies valid results in a short time (in one day) and by the preventive screening of the high risk patient group gives theoretically a solution for the problem of early diagnosis. Further object of the invention was to set up a diagnostic method which reduces the high number of false positive results caused by cross contaminations.

### Description of the invention

Surprisingly present inventors found that the *facC* gene coding for the extracellular pleiotrop autoregulator protein factor C isolated from *Streptomyces griseus* 45H (later identified as a member of the species *Streptomyces albidoflavus* and therefore named *Streptomyces albidoflavus* 45H), is present in the species *Aspergillus fumigatus, Neosartorya fischeri, Aspergillus terreus, Chaetomium globosum* and also in *Podospora anserina* (Biró et al., 1980; Birkó et al., 1999; Kiss et al., 2008). This finding is especially surprising because these fungi species are highly distant relatives of streptomycetes. It was also recognized, that the adequate part of the homologous *facC* gene is suitable for the exact identification of certain members of *Aspergillus* genus realizing much less false positive results, than in the case of any so far known methods. The reduction of the number of false positive results may be explained on one hand by the rare presence of the investigated *facC* gene in fungi, on the other hand by the relative high guanine and cytosine (G+C) content of the gene.

The invention relates to oligonucleotides for the detection of *Aspergillus* species. The homologues of *facC* gene of *Streptomyces griseus* in the species of *Aspergillus* genus afford the development of oligonucleotide probes that are specific for different *Aspergillus* species, preferably for pathogenic ones, like *A. fumigatus* and *A. terreus.* Hereby the invention offers a better solution than the previously available methods for the diagnosis of infection caused by *Aspergillus* and for species specific detection of these pathogens.

The invention relates to oligonucleotides which is specific for species in *Aspergillus* genus, and is able to hybridize under stringent conditions to a gene of a fungus species of the Aspergillus genus, said gene being a homologue of *facC* gene of *Streptomyces griseus* 45H. The sequence of said homologous gene is identical to any of SEQ ID NOs 118 to 120.

According to the invention the meaning of the "functional derivative" of an oligonucleotide is an oligonucleotide which contains no more than 1-2 added, substituted, deleted or inserted nucleotides compared to the sequence of the oligonucleotide according to the invention and the G+C ratio of said homologue does not differ with more than 10%, preferably does not differ with more than 5% compared to the oligonucleotide according to the invention, and in addition said homologue is suitable for detection and/or identification of fungi that cause aspergillosis.

The oligonucleotides according to the invention are suitable for the detection and/or identification of fungi preferably *Aspergillus fumigatus* or *Aspergillus terreus* that cause aspergillosis.

Since the oligonucleotides according to the invention are not based on ribosomal RNA (rRNA), these oligonucleotides may be used to verify identification methods based on the detection of rRNA. Furthermore if both the method according to the invention based on *facC* detection and an rRNA based method is performed this combined identification further improves the identification of Aspergillus species.

Additionally the invention relates to an *in vitro* diagnostic method for detection or identification of fungi species preferably *Aspergillus fumigatus* or *Aspergillus terreus* capable of causing aspergillosis, characterized by
a. isolating DNA isolation from a biological sample of a patient, said sample presumably containing fungi cells and wherein preferably said sample is blood, tissue, bronchoalveolar lavage or sputum;
b. amplifying DNA whereby amplifying a gene segment capable of hybridizing under stringent conditions to an oligonucleotide according to any of SEQ ID NOs 3, 6, 9, 12, 15, 18, 21, 24, 27, 30, 33, 36, 39, 42, 45, 48, 51, 54, 57, 60, 63, 66, 69, 72, 75, 78, 81, 84, 87, 90, 93, 96, 99, 102, 105, 108, 111, 114 or 117 in the presence of a fungus species capable of causing aspergillosis;
c. establishing fungi infection by the identification of the amplified gene segment.

According to a preferred embodiment of the invention in said method besides the above described gene segment an rRNA segment is also amplified.

According to the invention the term "stringent condition" means such hybridizing and then washing conditions that an ordinary person skilled in the art traditionally considers stringent. In detail hybridization under stringent conditions means that the temperature and the ionic strength is chosen in such a way that hybridization between two complementary DNA fragment is possible. For further definition of stringent conditions see the manual of Sambrook et al. (Sambrook, J. C., Fritsch, E. F. and Maniatis, T., 1989, "Molecular Cloning: A Laboratory Manual", Cold Spring Harbor Laboratory Press, Cold Spring Harbor, NY). A person skilled in the art will readily recognize that stringent conditions depend on the length (e.g. 10 to 40 base) of DNA sequences, primers, oligonucleotide probes or mixed oligonucleotide probes.

Without limitation a preferred example of stringent conditions is a reaction condition (temperature, composition of the solution etc.) under which the primers and the probe can hybridize with the target sequence only if they show 100% complementarity.

According to an advantageous embodiment of the present invention PCR is performed in step (b), or more preferably real-time quantitative PCR is performed. The real-time quantitative PCR can be performed advantageously with the following oligonucleotides according to the invention:
*Aspergillus fumigatus* assays in conventional 5'>3' orientation:

| | |
|---|---|
| SEQ ID NO 1: CAAAGTCGGCAGCCTTCTG | (19 mer) |
| SEQ ID NO 2: TGTCGCGATGCCAAAGGT | (18mer) |
| SEQ ID NO 3: CCGCATTGCTCTGG | (14mer) |
| | |
| SEQ ID NO 4: CCTCATCCAAACGCTTCGA | (19 mer) |
| SEQ ID NO 5: AGGGCTTTGTGACGGTAGAGATC | (23 mer) |
| SEQ ID NO 6: CTCTCTGCCCCCTCC | (15 mer) |
| | |
| SEQ ID NO 7: GAAACAGCGGGCGACCTAA | (19 mer) |
| SEQ ID NO 8: CCGACGTAGTTGCCGTCAA | (19 mer) |
| SEQ ID NO 9: ATCACCCAGCTCGAC | (15 mer) |
| | |
| SEQ ID NO 10: CAGCGGGCGACCTAACAAT | (19 mer) |
| SEQ ID NO 11: GGTACATGTGTCCGACGTAGTTG | (23 mer) |
| SEQ ID NO 12: CCCAGCTCGACTTT | (14 mer) |
| | |
| SEQ ID NO 13: CAACTACGTCGGACACATGTACCTA | (25 mer) |
| SEQ ID NO 14: TGCGCGCCGAAGGA | (14 mer) |
| SEQ ID NO 15: AGAGCTTTGGTCATGGC | (17 mer) |
| | |
| SEQ ID NO 16: CTCCGCTCAACTGGCAAAG | (19 mer) |
| SEQ ID NO 17: TCAATGGCGCAGGTATGCT | (19 mer) |
| SEQ ID NO 18: TCAAGCCCGTCGCCGA | (16 mer) |
| | |
| SEQ ID NO 19: AGCATACCTGCGCCATTGA | (19 mer) |
| SEQ ID NO 20: GCTGAGGTGATACCGGACGAT | (21 mer) |
| SEQ ID NO 21: CGGTGTACAACCGGCT | (16 mer) |
| | |
| SEQ ID NO 22: CGGTGTACAACCGGCTGATC | (20 mer) |
| SEQ ID NO 23: CATACACGGCGATATGCTTTGA | (22 mer) |
| SEQ ID NO 24: TCCGGTATCACCTCAGC | (17 mer) |
| | |
| SEQ ID NO 25: CAAGCAGCCGGAGTTGGA | (18 mer) |
| SEQ ID NO 26: ACTGTCCATACGCTGCATAACC | (22 mer) |
| SEQ ID NO 27: ACGCTGTCGTTGACTTT | (17 mer) |
| | |
| SEQ ID NO 28: GGTTATGCAGCGTATGGACAGTAT | (24 mer) |
| SEQ ID NO 29: CCGCTGGCCGCATATG | (16 mer) |
| SEQ ID NO 30: ATCTGCTGACGGGAAC | (16 mer) |
| | |
| SEQ ID NO 31: AATCCCCGACTCTCCACGAT | (20 mer) |
| SEQ ID NO 32: TCCGCCAGAGGTCATACGA | (19 mer) |
| SEQ ID NO 33: CGACCTCACCAAACC | (15 mer) |
| | |
| SEQ ID NO 34: GCCCTCGACTCAGGAGACTTG | (21 mer) |
| SEQ ID NO 35: CGAGGACCTTTCCGGAGAA | (19 mer) |
| SEQ ID NO 36: CAATCAGCCAACTCGA | (16 mer) |
| | |
| SEQ ID NO 37: CGCCGCGGAACCAAT | (15 mer) |
| SEQ ID NO 38: CTGCAGCGTCGGTTTCACT | (19 mer) |
| SEQ ID NO 39: AAGCGATACGTATATCTG | (18 mer) |
| | |
| SEQ ID NO 40: GCCACAATTGACCCCGTTTA | (20 mer) |
| SEQ ID NO 41: CACGGTCCCCGTCTGGTAT | (19 mer) |
| SEQ ID NO 42: AGCGGTTGATCGTGC | (15 mer) |
| | |
| SEQ ID NO 43: GCGAGCGCAGGCAATTT | (17 mer) |
| SEQ ID NO 44: CCTTGACGAGATGCGGAATC | (20 mer) |
| SEQ ID NO 45: TCGAACCCACTGGCC | (15 mer) |
| SEQ ID NO 46: AGCGCAGGCAATTTTTCG | (18 mer) |
| SEQ ID NO 47: CCTTGACGAGATGCGGAATC | (20 mer) |
| SEQ ID NO 48: ACCCACTGGCCAAAT | (15 mer) |
| | |
| SEQ ID NO 49: TCGAACCCACTGGCCAAAT | (19 mer) |
| SEQ ID NO 50: CTTTGCCCCGAGCTCCTT | (18 mer) |
| SEQ ID NO 51: AGATTCCGCATCTCGT | (16 mer) |
| | |
| SEQ ID NO 52: GGACTTGAATACTGGGAAGCTTGT | (24 mer) |
| SEQ ID NO 53: CGTCGACCCCGCCTTT | (16 mer) |
| SEQ ID NO 54: CAAGGGCCTGTCACC | (15 mer) |
| | |
| SEQ ID NO 55: GAGCTGAAACTGCCGTTGATG | (21 mer) |
| SEQ ID NO 56: CCTCTGCGGTACTGGTTTCG | (20 mer) |
| SEQ ID NO 57: AGCCTGTGTACATGGAT | (17 mer) |
| | |
| SEQ ID NO 58: CGTTGATGCAGCCTGTGTACAT | (22 mer) |
| SEQ ID NO 59: GACGCGCGGCTTCCT | (15 mer) |
| SEQ ID NO 60: TTCTCGAAACCAGTACCGC | (19 mer) |
| | |
| SEQ ID NO 61: GATGCAGCCTGTGTACATGGAT | (22 mer) |
| SEQ ID NO 62: GACGCGCGGCTTCCT | (15 mer) |
| SEQ ID NO 63: TCTCGAAACCAGTACCGCA | (19 mer) |
| | |
| SEQ ID NO 64: CCTCGTATGGACGCGGTAA | (19 mer) |
| SEQ ID NO 65: GACTCCACTATGGCCTGCTAGTC | (23 mer) |
| SEQ ID NO 66: TGGGCCACATGTTG | (14 mer) |
| | |
| SEQ ID NO 67: CCAATACGTCGCATACCTGTCA | (22 mer) |
| SEQ ID NO 68: GGCGACCGGCGTATACTTC | (19 mer) |
| SEQ ID NO 69: AGACATAGACGACTGCCCT | (19 mer) |
| | |
| SEQ ID NO 70: AGCGGCGCCTTGGTTAG | (17 mer) |
| SEQ ID NO 71: CGACGTCGCAGCCAAAGT | (18 mer) |
| SEQ ID NO 72: TTCGCTATGCATATAGACCT | (20 mer) |
| | |
| SEQ ID NO 73: GTTCCTCGAGCCCCGTTT | (18 mer) |
| SEQ ID NO 74: TGTATGGCGGGCATTCG | (17 mer) |
| SEQ ID NO 75: CAATTGCAGAAAGTCCCTATA | (21 mer) |
| | |
| SEQ ID NO 76: TCGAGCCCCGTTTCCAA | (17 mer) |
| SEQ ID NO 77: TGTATGGCGGGCATTCG | (17 mer) |
| SEQ ID NO 78: TGCAGAAAGTCCCTATATG | (19 mer) |
| | |

| *Aspergillus terreus* assays in conventional 5'>3' orientation: | |
|---|---|
| SEQ ID NO 79: CTCCTCGCATCCAGCGTAAG | (20 mer) |
| SEQ ID NO 80: CAGGTCGAATTGGGAAGAAGAC | (22 mer) |
| SEQ ID NO 81: TTGGGCGGCGCTAC | (14 mer) |
| | |
| SEQ ID NO 82: TCTGTGTATCACCCAGCTTGATTT | (24 mer) |
| SEQ ID NO 83: AACCCGATCAGGTCCATTGA | (20 mer) |
| SEQ ID NO 84: CACGGAAACATTGTCGG | (17 mer) |
| | |
| SEQ ID NO 85: CGAATGGATACGGGAAGAAGCT | (22 mer) |
| SEQ ID NO 86: CGAGCGAGGCATCGGTATG | (19 mer) |
| SEQ ID NO 87: TTGCCATTGACAAACTT | (17 mer) |
| | |
| SEQ ID NO 88: GGCAAAACTCTGTCGCATACC | (21 mer) |
| SEQ ID NO 89: GCACCCTCAACAGCAGTGAAT | (21 mer) |
| SEQ ID NO 90: ATGCCTCGCTCGCGA | (15 mer) |
| | |
| SEQ ID NO 91: CTGCTCTATTGACCCGGTGAA | (21 mer) |
| SEQ ID NO 92: TTTTTCCCGCTCAGAGCATATC | (22 mer) |
| SEQ ID NO 93: ACCGGCTCGTGGTC | (14 mer) |
| | |
| SEQ ID NO 94: GCATTGCCGTGTTCGATCT | (19 mer) |
| SEQ ID NO 95: ATCCGCCAGTTTCGTAGAAAAG | (22 mer) |
| SEQ ID NO 96: CGCAACAAAGGGTG | (14 mer) |
| | |
| SEQ ID NO 97: AACTGGCGGATATCGCTCAT | (20 mer) |
| SEQ ID NO 98: AGCATAGCCCTGGAACACCTT | (21 mer) |
| SEQ ID NO 99: CTTCGCTGGATACGCTAT | (18 mer) |
| | |
| SEQ ID NO 100: GCTTCTGGTGGTGTGGTCAA | (20 mer) |
| SEQ ID NO 101: GACCACCTTCCCAGTATTCAAGTC | (24 mer) |
| SEQ ID NO 102: CGCAGGTGACCGCC | (14 mer) |
| | |
| SEQ ID NO 103: CCGTTTTGAGACAGCGTATGG | (21 mer) |
| SEQ ID NO 104: ACGTGGGAGTTGTCGTCACTT | (21 mer) |
| SEQ ID NO 105: TACGGAGCGAGCGCT | (15 mer) |
| | |
| SEQ ID NO 106: TTCGCGTAACGGCACAAG | (18 mer) |
| SEQ ID NO 107: GGCGGTCAAAGCATCTTTTC | (20 mer) |
| SEQ ID NO 108: ACCCCCGGCGTTGT | (14 mer) |
| | |
| SEQ ID NO 109: CGCGTAACGGCACAAGCTA | (19 mer) |
| SEQ ID NO 110: GGCGGTCAAAGCATCTTTTC | (20 mer) |
| SEQ ID NO 111: ACCCCCGGCGTTGT | (14 mer) |
| | |
| SEQ ID NO 112: TAGACCCCCGGCGTTGTT | (18 mer) |
| SEQ ID NO 113: CCTACTCGCTATAGGCGGTCAA | (22 mer) |
| SEQ ID NO 114: CCCACTGAAAAGATG | (15 mer) |
| | |
| SEQ ID NO 115: GATAGAAGAATGCCCTCTCAGCAT | (24 mer) |
| SEQ ID NO 116: CGCCAGTGGACGCTCAAC | (18 mer) |
| SEQ ID NO 117: CGACGAAGACCACCACA | (17 mer). |

| | |
|---|---|
| Note: in the grouped sequences of three, the first is the forward primer, the second is the reverse primer and the third is the probe. | |

In step c) of the above method fluorescent dye or a method based on hydrolysis or hybridisation probes may be used for the identification of the amplified gene-part.

Furthermore the invention relates to a diagnostic kit for the specific identification of *Aspergillus* fungi from biological samples, which kit contains the oligonucleotide or its functional derivatives according to the invention.

### Brief description of the drawings

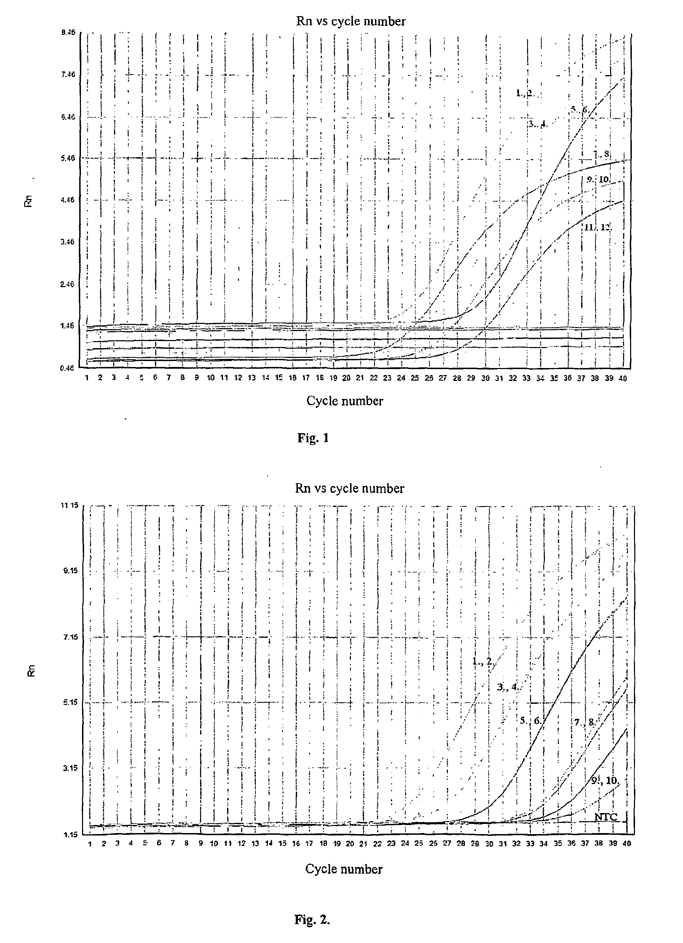

**Figure 1**: Normalized fluorescent values (rn) of *Aspergillus fumigatus* and *A. terreus* assays as function of the number of cycles using the indicated amount of template DNA.
   In case of using *A. fumigatus* template and *A. fumigatus* specific assay curves 1, 2 (4 ng), curves 3, 4 (0.8 ng), and curves 5, 6 (0.16 ng). In case of *A. terreus* template and *A. terreus* assay curves 7, 8, 9, 10, and 11, 12 using the same amount of template, respectively, show the amplification. The *A. fumigatus* TaqMan assay with *A. terreus* DNA or vice versa gave no amplification.
**Figure 2****:** Determination of sensitivity of *Aspergillus fumigatus* TaqMan assay.
   Normalised fluorescent values as function of cycle numbers are showed on curves 1, 2 using 4 ng, 3, 4 0.8 ng, 5, 6 0.16 ng, 7, 8 16 pg, and 9, 10 0.16 pg template DNA. NTC (control without template)
**Figure 3****:** Determination of sensitivity of *Aspergillus terreus* TaqMan assay.
   Normalised fluorescent values as function of cycle numbers are showed on curves 1, 2 using 4 ng, 3, 4 0.8 ng, 5, 6 0.16 ng, 7, 8 16 pg, and 9, 10 0.16 pg template DNA. NTC (control without template)
**Figure 4****:** Application of *Aspergillus fumigatus* specific assay on human genomic template DNA originated from three different healthy persons. Curves 1 and 2, 3 and 4, 5 and 6, show the results of the parallel measurements of the same DNA samples. Amount of DNA is 30 ng in each case. Curves 7 and 8 are control without template DNA. Relative fluorescence (rn) does not change significantly as a function of the number of cycles, so human DNA does not give fals positive result.

### Examples

### Example 1

### Detection of Aspergillus fumigatus DNA in biological sample

According to standard methods described in literature, fungi DNA was isolated from blood, bronchoalveolar lavage, sputum, tissue etc. and DNA concentration was measured.

Measurement was performed in 25 µl final sample volume composed of:
12.5 µl TaqMan^{®} Universal PCR Master Mix (2X) (Applied Biosystems part number 4324018).
1.25 µl TaqMan^{®} gene expression assay mix (20X) (Applied Biosystems part number 4332078) containing the forward (SEQ ID NO: 25) and reverse (SEQ ID NO: 26) primers furthermore the FAM (6-carboxilfluoresceine) labeled TaqMan-MGB ("Minor Groove Binder") probe (SEQ ID NO: 27) that is specific for the investigated gene. Primers and probe were delivered by Applied Biosystems.
2.5 µl tested DNA sample,
8.75 µl nuclease free water.

Measurement was performed by Applied Biosystems 7500 Real Time PCR equipment applying the following reaction parameters:
Part 1: denaturing DNA, 95°C, 10 min.
Part 2: repeating 40x the following steps:
   Step 1: 95°C, 15 seconds
   Step 2: 60°C, 60 seconds
   Step 3: measurement of fluorescence each time after the Step 2.

In case of negative control (NTC, non-template control) 5 µl distilled water was applied instead of the examined sample.

### Example 2

### Detection of Aspergillus terreus DNA in biological sample

According to standard methods described in literature, fungi DNA was isolated from blood, bronchoalveolar lavage, sputum, tissue etc. and DNA concentration was established.

Measurement was performed in 25 µl final sample volume composed of:
12.5 µl TaqMan^{®} Universal PCR Master Mix (2X) (Applied Biosystems part number 4324018).
1.25 µl TaqMan^{®} gene expression assay mix (20X) (Applied Biosystems part number 4332078) containing the forward (SEQ ID NO: 85) and reverse (SEQ ID NO: 86) primers furthermore the FAM (6-carboxilfluoresceine) labeled TaqMan-MGB ("Minor Groove Binder") probe (SEQ ID NO: 87) that is specific for the investigated gene. Primers and probe was delivered by the Applied Biosystems.
2.5 µl tested DNA sample,
8.75 µl nuclease free water.

Measurement was performed by Applied Biosystems 7500 Real Time PCR equipment applying the following reaction parameters:
Part 1: denaturing DNA, 95°C, 10 min.
Part 2: repeating 40x the following steps:
   Step 1: 95°C, 15 seconds
   Step 2: 60°C, 60 seconds
   Step 3: measurement of fluorescence each time after step 2.
In case of negative control (NTC, non-template control) 5 µl distilled water was applied instead of the examined sample.

On the basis of the above described example 1 and 2 it was demonstrated that *Aspergillus fumigatus* assay gives signal only with *Aspergillus fumigatus* DNA and not with *Aspergillus terreus* DNA, furthermore *Aspergillus terreus* assay gives signal only in case of *Aspergillus terreus* DNA and not with *Aspergillus fumigatus* DNA (Fig. 1).

### Example 3

### Determination of the sensitivity of Aspergillus fumigatus TaqMan assay.

To determine the sensitivity of the assay applying the oligonucleotides according to the invention the method according to example 1 was repeated with different amount of DNA templates. Figure 2 shows the sensitivity of *Aspergillus fumigatus* TaqMan assay. The following amounts of template DNA were used: curves 1, 2 applying 4 ng, 3, 4 0.8 ng, 5, 6 0.16 ng, 7, 8 16 pg, and 9, 10 0.16 pg template DNA. According to the figure it can be seen that the sensitivity of the assay is between about 10 and about 200 femtograms, more preferably between about 16 and about 160 femtograms.

### Example 4

### Determination of the sensitivity of Aspergillus terreus TaqMan assay.

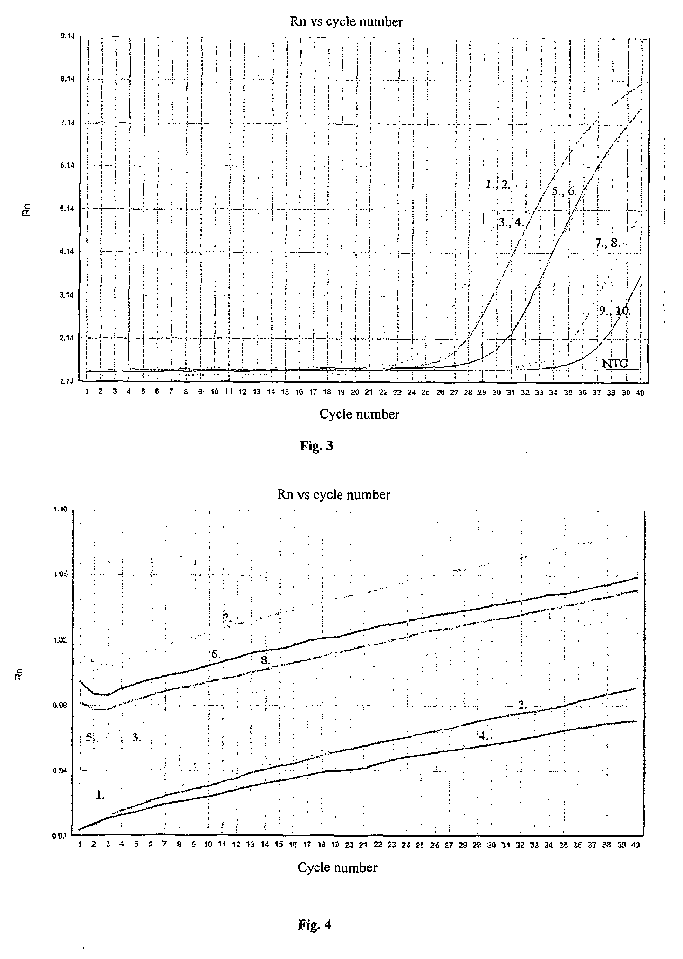

To determine the sensitivity of the measurement methods applying the oligonucleotides described by the invention the method according to example 1 was repeated with different amount of DNA templates. Figure 3 shows the sensitivity of *Aspergillus terreus* TaqMan assay. The following amounts of template DNA were used: curves 1 and 2 applying 4 ng, 3, 4 0.8 ng, 5, 6 0.16 ng, 7, 8 16 pg, and 9, 10 0.16 pg template DNA. According to the figure it can be seen that the sensitivity of the assay is between about 10 and about 200 femtograms, more preferably between about 16 and about 160 femtograms.

### Example 5

Use of assays specific for *Aspergillus fumigatus* and *Aspergillus terreus* on human genomic DNA template. Assays based on probes according to the invention were investigated for cross-reactions with human DNA. Genomic DNA was isolated from blood samples of three different donors and were used as templates. Neither *Aspergillus fumigatus* (Figure 4.) nor *Aspergillus terreus* assay gave any signal with the human DNA samples. As it can be seen on the figure, the relative fluorescence did not change significantly even during 40 cycles.

### References

Abbott SP et al., (1995) Fatal Cerebral Mycoses Caused by the Ascomycete Chaetomium Strumarium. Journal of Clinical Microbiology 10: 2692-2698.
Anandi V et al., (1989) Cerebral Phaeohyphomycosis Caused by Chaetomium globosum in a Renal Transplant Recipient. Journal of Clinical Microbiolog 27(10): 2226-2229.
Aquino VR et al., (2007) Update on the contribution of galactomannan for the diagnosis of invasive aspergillosis. Mycopathologia 163: 191-202.
Biró S et al., (1980) A substance effecting differentiation in Streptomyces griseus. European Journal of biochemistry/FEBS 103 (2): 359-363.
Birkó Z et al., (1999) Characterization of the gene for factor C, an extracellular signal protein involved in morphological differentiation of Streptomyces griseus. Microbiology 145(9): 2245-2253
Bolehovska R et al., (2006) Detection of Aspergillus spp. in biological samples by real-time PCR. Biomed Pap Med Fac Univ Palacky Olomouc Czech Repub 150 (2): 245-248.
Cesaro S (2008) Assessment of the lightcycler PCR assay for diagnosis of invasive aspergillosis in paediatric patients with onco-haematological diseases. Mycoses 51(6):497-504. Epub 2008 Mar 3.
Denning DW (2006) Aspergillosis. Educational material supplied by The Aspergillus Website. Shering-Plough Corporation*.*
Donnelly JP (2006) Polymerase chain reaction for diagnosing invasive aspergillosis: getting closer but still a way to go. Clin Infect Dis 42: 487-9.
Erjavec Z and Verweij PE (2002) Recent progress in the diagnosis of fungal infections in the immunocompromised host. Drug Resistance Updates. 5 (1): 3-10.
Florent M et al., (2006) Prospective Evaluation of a Polymerase Chain Reaction-ELISA Targeted to Aspergillus fumigatus and Aspergillus flavus for the Early Diagnosis of Invasive Aspergillosis in Patients with Hematological Malignancies. The Journal of Infectious Diseases 193: 741-7.
Guarro J et al., (2002) Cerebral Aspergillosis Caused by Neosartorya hiratsukae, Brazil. Emerging Infectious Diseases 8(9):989-91*.*
Kiss Z et al., (2008) Streptomyces griseus 45H, a producer of the extracellular autoregulator protein factor C, is a member of the species Streptomyces albidoflavus. International Journal of Systematic and Evolutionary Microbiology 58: 1029-1031.
Hummel M et al., (2006) Detection of Aspergillus DNA in Cerebrospinal Fluid from Patients with Cerebral Aspergillosis by a Nested PCR Assay. Journal of Clinical Microbiology. 44(11): 3989-3993.
Pagano L et al., (2006) The epidemiology of fungal infections in patients with hematologic malignancies: the Sefem-2004 study. Haematologica 91(8):1068-75.
Sambrook, J. C. et al., (1989) Molecular Cloning: A Laboratory Manual, ed: Cold Spring Harbor Laboratory Press, Cold Spring Harbor, N. Y
Segal BH and Walsh TJ. (2006) Current approaches to diagnosis and treatment of invasive aspergillosis. American Journal of respiratory and critical care medicine 173(7): 707-17*.*
Vidal JE et al., (2005) Cerebral Aspergillosis due to Aspergillus fumigatus in AIDS patient: First Culture-proven case reported in Brasil. Revista do Instituto de Medicina Tropical de Säo-Paulo 47 (3): 161-165.
Vonberg, R. P. and Gastmeier, P. (2006) Nosocomial aspergillosis in outbreak settings. Journal of Hospital Infection 63: 246-254.
White PL et al., (2006a) A consensus on fungal polymerase chain reaction diagnosis: a United Kingdom-Ireland evaluation of polymerase chain reaction methods for detection of systemic fungal infections. The Journal of Molecular Diagnosis. 8 (3): 376-384.
White PL et al., (2006b) The Evolution of a Whole Blood Polymerase Chain Reaction Assay for the Detection of Invasive Aspergillosis in Hematology Patients in a Routine Clinical Setting. Clinical Infectious Diseases 42(4): 487-9.
White PL and Barnes RA (2006) Aspergillus PCR-platforms, strengths and weaknesses. Med Mycol 44: S191-8.
Williamson EC et al., (2000) Aspergillus antigen testing in bone marrow transplant recipients. Journal of Clinical Pathology. 53 (5): 362-366.
Yeghen T et al., (1996) Chaetomium pnemonia in patient with acute myeloid leukaemia. Journal of clinical pathology 49:184-186.

## Claims

1. An oligonucleotide specific for fungi species of *Aspergillus* genus which is able to hybridize under stringent conditions to a gene of a fungus species of the Aspergillus genus, said gene being a homologue of *Streptomyces griseus* 45H C factor gene, wherein the sequence of said homologue gene is identical to any of SEQ ID NO 118 to 120.

2. The oligonucleotide according to claim 1 *which* is 12 to 27, preferably 14 to 25 nucleotide in length.

3. The oligonucleotide according to claim 1 or 2, wherein the fungi species of *Aspergillus genus* is a pathogenic *Aspergillus* species preferably *Aspergillus fumigatus* or *Aspergillus terreus.*

4. The oligonucleotide according to any of claims 1 to 3 the sequence of which is any of SEQ ID NO 1 to 117 or a functional derivative thereof.

5. Use of an oligonucleotide according to any of claims 1 to -4 for the detection and/or identification of fungi species capable of causing aspergillosis.

6. Use of an oligonucleotide according to any of claims 1 to 4 for the identification of *Aspergillus fumigatus,* wherein the sequence of the oligonucleotide is any of SEQ ID NO 1 to 78 or a functional derivative thereof.

7. Use of an oligonucleotide according to any of claims 1 to 4 for the identification of *Aspergillus terreus,* wherein the sequence of the oligonucleotide is any of SEQ ID NO 79 to 117 or a functional derivative thereof.

8. An *in vitro* diagnostic method for detection and/or identification of fungi species capable of causing aspergillosis **characterized by**:
a) isolating DNA isolation from a biological sample of a patient, said sample presumably containing fungi cells and wherein preferably said sample is blood, tissue, bronchoalveolar lavage or sputum;
b) amplifying DNA whereby amplifying a gene segment capable of hybridizing under stringent conditions to an oligonucleotide according to any of SEQ ID NO 3, 6, 9, 12, 15, 18, 21, 24, 27, 30, 33, 36, 39, 42, 45, 48, 51, 54, 57, 60, 63, 66, 69, 72, 75, 78, 81, 84, 87, 90, 93, 96, 99, 102, 105, 108, 111, 114 or 117 in the presence of a fungus species capable of causing aspergillosis;
c) establishing fungi infection by the identification of the amplified gene segment.

9. The method according to claim 8, wherein the fungus species causing aspergillosis is *Aspergillus fumigatus* or *Aspergillus terreus*

10. The method according to claim 8 or 9, wherein in step (b) PCR or preferably quantitative real-time PCR is performed.

11. The method according to any of claims 8 to 10, wherein in step (c) fluorescent dye or method based on hydrolysis or hybridization probes is used to identify the amplified gene segment.

12. A diagnostic kit for specific identification of *Aspergillus* fungi species from biological samples, said kit containing an oligonucleotide or its functional derivative according to any of claims 1 to 4.

## Patentansprüche

1. Ein Oligonukleotid spezifisch für eine Pilzart der Gattung *Aspergillus,* die unter stringenten Bedingungen in der Lage ist an ein Gen einer Pilzart der Gattung *Aspergillus* zu hybridisieren, wobei das Gen ein Homolog des *Streptomyces griseus* 45H C Faktor Gens ist, wobei die Sequenz des homologen Gens identisch ist zu einer der Sequenzen von SEQ ID NO. 118 bis 120.

2. Das Oligonukleotid nach Anspruch 1, das 12 bis 27 Nukleotide, bevorzugt 14 bis 25 Nukleotide, lang ist.

3. Das Oligonukleotid nach Anspruch 1 oder 2, wobei die Pilzart der Gattung *Aspergillus* eine pathogene *Aspergillus* Art ist, bevorzugt *Aspergillus fumigatus* oder *Aspergillus terreus.*

4. Das Oligonukleotid nach einem der Ansprüche 1 bis 3, wobei die Sequenz eine der Sequenzen von SEQ ID NO. 1 bis 117 ist oder ein funktionelles Derivat davon.

5. Verwendung eines Oligonukleotids nach einem der Ansprüche 1 bis 4 zum Nachweis und/oder zur Identifikation einer Pilzart, die Aspergillose auslösen kann.

6. Verwendung eines Oligonukleotids nach einem der Ansprüche 1 bis 4 zur Identifikation von *Aspergillus fumigatus,* wobei die Sequenz des Oligonukleotides eine der Sequenzen von SEQ ID NO. 1 bis 78 oder ein funktionelles Derivat davon ist.

7. Verwendung eines Oligonukleotides nach einem der Ansprüche 1 bis 4 zur Identifikation von *Aspergillus terreus,* wobei die Sequenz des Oligonukleotides eine der Sequenzen von SEQ ID NO. 79 bis 117 oder ein funktionelles Derivat davon ist.

8. Ein *in vitro* Diagnoseverfahren zum Nachweis und/oder zur Identifikation einer Pilzart, die Aspergillose auslösen kann, **gekennzeichnet durch**:
a) Isolieren einer DNA-Isolierung aus einer biologischen Probe eines Patienten, wobei die Probe voraussichtlich Pilzzellen enthält und wobei die Probe bevorzugt Blut, Gewebe, bronchoalveoläre Lavage oder Sputum ist;
b) Amplifizieren von DNA, wodurch ein Gensegment amplifiziert wird, das in der Lage ist unter stringenten Bedingungen an ein Oligonukleotid gemäß einer der Sequenzen von SEQ ID NO. 3, 6, 9, 12, 15, 18, 21, 24, 27, 30, 33, 36, 39, 42, 45, 48, 51, 54, 57, 60, 63, 66, 69, 72, 75, 78, 81, 84, 87, 90, 93, 96, 99, 102, 105, 108, 111, 114 oder 117 zu hybridisieren, in der Gegenwart einer Pilzart, die Aspergillose auslösen kann.
c) Feststellen einer Pilzinfektion **durch** die Identifikation des amplifizierten Gensegments.

9. Das Verfahren nach Anspruch 8, wobei die Pilzart, die Aspergillose auslöst, *Aspergillus fumigatus* oder *Aspergillus terreus* ist.

10. Das Verfahren nach Anspruch 8 oder 9, wobei in Schritt (b) eine PCR, bevorzugt eine quantitative real-time PCR, durchgeführt wird.

11. Das Verfahren nach einem der Ansprüche 8 bis 10, wobei in Schritt (c) ein fluoreszierender Farbstoff oder ein Verfahren basierend auf Hydrolyse oder Hybridisationssonden zur Identifikation des amplifizierten Gensegments verwendet wird.

12. Ein diagnostisches Kit zur spezifischen Identifikation von *Aspergillus* Pilzarten aus biologischen Proben, wobei das Kit ein Oligonukleotid oder eines seiner funktionellen Derivate nach einem der Ansprüche 1 bis 4 enthält.

## Revendications

1. Oligonucléotide spécifique pour l'espèce de champignons du genre *Aspergillus,* qui est capable de s'hybrider dans des conditions strictes à un gène d'une espèce de champignon du genre *Aspergillus,* ledit gène étant un homologue du gène de facteur 45H C *Streptomyces griseus,* dans lequel la séquence dudit gène homologue est identique à n'importe laquelle des SEQ. ID N°118 à 120.

2. Oligonucléotide selon la revendication 1, qui est de 12 à 27, de préférence de 14 à 25 nucléotides de long.

3. Oligonucléotide selon la revendication 1 ou 2, dans lequel l'espèce de champignon du genre *Aspergillus* est une espèce *Aspergillus* pathogène, de préférence un *Aspergillus fumigatus* ou un *Aspergillus terreus.*

4. Oligonucléotide selon l'une quelconque des revendications 1 à 3, dont la séquence est n'importe laquelle des SEQ. ID N°1 à 117 ou un dérivé fonctionnel correspondant.

5. Utilisation d'un oligonucléotide selon l'une quelconque des revendications 1 à 4 pour la détection et/ou l'identification de l'espèce de champignons capable de provoquer l'aspergillose.

6. Utilisation d'un oligonucléotide selon l'une quelconque des revendications 1 à 4 pour l'identification de *l'Aspergillus fumigatus,* dans lequel la séquence de l'oligonucléotide est n'importe laquelle des SEQ. ID N°1 à 78 ou un dérivé fonctionnel correspondant.

7. Utilisation d'un oligonucléotide selon l'une quelconque des revendications 1 à 4 pour l'identification de *l'Aspergillus terreus,* dans lequel la séquence de l'oligonucléotide est n'importe laquelle des SEQ. ID N°79 à 117 ou un dérivé fonctionnel correspondant.

8. Procédé de diagnostic *in vitro* pour la détection et/ou l'identification d'espèces de champignons capables de provoquer une aspergillose, **caractérisé par** :
a) l'isolation de l'ADN d'un échantillon biologique d'un patient, ledit échantillon contenant probablement des cellules de champignons et dans lequel de préférence ledit échantillon est un échantillon de sang, de tissu, de lavage broncho-alvéolaire ou de crachat ;
b) l'amplification d'ADN, en amplifiant ainsi un segment génique capable de s'hybrider dans des conditions strictes à un oligonucléotide selon l'une quelconque des SEQ. ID N°3, 6, 9, 12, 15, 18, 21, 24, 27, 30, 33, 36, 39, 42, 45, 48, 51, 54, 57, 60, 63, 66, 69, 72, 75, 78, 81, 84, 87, 90, 93, 96, 99, 102, 105, 108, 111, 114 ou 117 en présence d'une espèce de champignon capable de provoquer une aspergillose ;
c) l'établissement d'une infection fongique par l'identification du segment génique amplifié.

9. Procédé selon la revendication 8, dans lequel l'espèce de champignon provoquant l'aspergillose est un *Aspergillus fumigatus* ou un *Aspergillus terreus.*

10. Procédé selon la revendication 8 ou 9, dans lequel, à l'étape (b), une PCR, ou de préférence une PCR quantitative en temps réel, est réalisée.

11. Procédé selon l'une quelconque des revendications 8 à 10, dans lequel, à l'étape (c), un colorant fluorescent ou un procédé à base d'hydrolyse ou de sondes d'hybridation est utilisé pour identifier le segment génique amplifié.

12. Kit de diagnostic pour une identification spécifique de l'espèce de champignon *Aspergillus* à partir d'échantillons biologiques, ledit kit contenant un oligonucléotide ou son dérivé fonctionnel selon l'une quelconque des revendications 1 à 4.


### SEQUENCE LISTING

<110> University of Debrecen
<120> OLIGONUCLEOTIDES FOR THE DETECTION OF ASPERGILLUS SPECIES
<130> P106688
<160> 120
<170> PatentIn version 3.5
<210> 1
   <211> 19
   <212> DNA
   <213> Aspergillus fumigatus
<400> 1
   caaagtcggc agccttctg 19
<210> 2
   <211> 18
   <212> DNA
   <213> Aspergillus fumigatus
<400> 2
   tgtcgcgatg ccaaaggt 18
<210> 3
   <211> 14
   <212> DNA
   <213> Aspergillus fumigatus
<400> 3
   ccgcattgct ctgg 14
<210> 4
   <211> 19
   <212> DNA
   <213> Aspergillus fumigatus
<400> 4
   cctcatccaa acgcttcga 19
<210> 5
   <211> 23
   <212> DNA
   <213> Aspergillus fumigatus
<400> 5
   agggctttgt gacggtagag atc 23
<210> 6
   <211> 15
   <212> DNA
   <213> Aspergillus fumigatus
<400> 6
   ctctctgccc cctcc 15
<210> 7
   <211> 19
   <212> DNA
   <213> Aspergillus fumigatus
<400> 7
   gaaacagcgg gcgacctaa 19
<210> 8
   <211> 19
   <212> DNA
   <213> Aspergillus fumigatus
<400> 8
   ccgacgtagt tgccgtcaa 19
<210> 9
   <211> 15
   <212> DNA
   <213> Aspergillus fumigatus
<400> 9
   atcacccagc tcgac 15
<210> 10
   <211> 19
   <212> DNA
   <213> Aspergillus fumigatus
<400> 10
   cagcgggcga cctaacaat 19
<210> 11
   <211> 23
   <212> DNA
   <213> Aspergillus fumigatus
<400> 11
   ggtacatgtg tccgacgtag ttg 23
<210> 12
   <211> 14
   <212> DNA
   <213> Aspergillus fumigatus
<400> 12
   cccagctcga cttt 14
<210> 13
   <211> 25
   <212> DNA
   <213> Aspergillus fumigatus
<400> 13
   caactacgtc ggacacatgt accta 25
<210> 14
   <211> 14
   <212> DNA
   <213> Aspergillus fumigatus
<400> 14
   tgcgcgccga agga 14
<210> 15
   <211> 17
   <212> DNA
   <213> Aspergillus fumigatus
<400> 15
   agagctttgg tcatggc 17
<210> 16
   <211> 19
   <212> DNA
   <213> Aspergillus fumigatus
<400> 16
   ctccgctcaa ctggcaaag 19
<210> 17
   <211> 19
   <212> DNA
   <213> Aspergillus fumigatus
<400> 17
   tcaatggcgc aggtatgct 19
<210> 18
   <211> 16
   <212> DNA
   <213> Aspergillus fumigatus
<400> 18
   tcaagcccgt cgccga 16
<210> 19
   <211> 19
   <212> DNA
   <213> Aspergillus fumigatus
<400> 19
   agcatacctg cgccattga 19
<210> 20
   <211> 21
   <212> DNA
   <213> Aspergillus fumigatus
<400> 20
   gctgaggtga taccggacga t 21
<210> 21
   <211> 16
   <212> DNA
   <213> Aspergillus fumigatus
<400> 21
   cggtgtacaa ccggct 16
<210> 22
   <211> 20
   <212> DNA
   <213> Aspergillus fumigatus
<400> 22
   cggtgtacaa ccggctgatc 20
<210> 23
   <211> 22
   <212> DNA
   <213> Aspergillus fumigatus
<400> 23
   catacacggc gatatgcttt ga 22
<210> 24
   <211> 17
   <212> DNA
   <213> Aspergillus fumigatus
<400> 24
   tccggtatca cctcagc 17
<210> 25
   <211> 18
   <212> DNA
   <213> Aspergillus fumigatus
<400> 25
   caagcagccg gagttgga 18
<210> 26
   <211> 22
   <212> DNA
   <213> Aspergillus fumigatus
<400> 26
   actgtccata cgctgcataa cc 22
<210> 27
   <211> 17
   <212> DNA
   <213> Aspergillus fumigatus
<400> 27
   acgctgtcgt tgacttt 17
<210> 28
   <211> 24
   <212> DNA
   <213> Aspergillus fumigatus
<400> 28
   ggttatgcag cgtatggaca gtat 24
<210> 29
   <211> 16
   <212> DNA
   <213> Aspergillus fumigatus
<400> 29
   ccgctggccg catatg 16
<210> 30
   <211> 16
   <212> DNA
   <213> Aspergillus fumigatus
<400> 30
   atctgctgac gggaac 16
<210> 31
   <211> 20
   <212> DNA
   <213> Aspergillus fumigatus
<400> 31
   aatccccgac tctccacgat 20
<210> 32
   <211> 19
   <212> DNA
   <213> Aspergillus fumigatus
<400> 32
   tccgccagag gtcatacga 19
<210> 33
   <211> 15
   <212> DNA
   <213> Aspergillus fumigatus
<400> 33
   cgacctcacc aaacc 15
<210> 34
   <211> 21
   <212> DNA
   <213> Aspergillus fumigatus
<400> 34
   gccctcgact caggagactt g 21
<210> 35
   <211> 19
   <212> DNA
   <213> Aspergillus fumigatus
<400> 35
   cgaggacctt tccggagaa 19
<210> 36
   <211> 16
   <212> DNA
   <213> Aspergillus fumigatus
<400> 36
   caatcagcca actcga 16
<210> 37
   <211> 15
   <212> DNA
   <213> Aspergillus fumigatus
<400> 37
   cgccgcggaa ccaat 15
<210> 38
   <211> 19
   <212> DNA
   <213> Aspergillus fumigatus
<400> 38
   ctgcagcgtc ggtttcact 19
<210> 39
   <211> 18
   <212> DNA
   <213> Aspergillus fumigatus
<400> 39
   aagcgatacg tatatctg 18
<210> 40
   <211> 20
   <212> DNA
   <213> Aspergillus fumigatus
<400> 40
   gccacaattg accccgttta 20
<210> 41
   <211> 19
   <212> DNA
   <213> Aspergillus fumigatus
<400> 41
   cacggtcccc gtctggtat 19
<210> 42
   <211> 15
   <212> DNA
   <213> Aspergillus fumigatus
<400> 42
   agcggttgat cgtgc 15
<210> 43
   <211> 17
   <212> DNA
   <213> Aspergillus fumigatus
<400> 43
   gcgagcgcag gcaattt 17
<210> 44
   <211> 20
   <212> DNA
   <213> Aspergillus fumigatus
<400> 44
   ccttgacgag atgcggaatc 20
<210> 45
   <211> 15
   <212> DNA
   <213> Aspergillus fumigatus
<400> 45
   tcgaacccac tggcc 15
<210> 46
   <211> 18
   <212> DNA
   <213> Aspergillus fumigatus
<400> 46
   agcgcaggca atttttcg 18
<210> 47
   <211> 20
   <212> DNA
   <213> Aspergillus fumigatus
<400> 47
   ccttgacgag atgcggaatc 20
<210> 48
   <211> 15
   <212> DNA
   <213> Aspergillus fumigatus
<400> 48
   acccactggc caaat 15
<210> 49
   <211> 19
   <212> DNA
   <213> Aspergillus fumigatus
<400> 49
   tcgaacccac tggccaaat 19
<210> 50
   <211> 18
   <212> DNA
   <213> Aspergillus fumigatus
<400> 50
   ctttgccccg agctcctt 18
<210> 51
   <211> 16
   <212> DNA
   <213> Aspergillus fumigatus
<400> 51
   agattccgca tctcgt 16
<210> 52
   <211> 24
   <212> DNA
   <213> Aspergillus fumigatus
<400> 52
   ggacttgaat actgggaagc ttgt 24
<210> 53
   <211> 16
   <212> DNA
   <213> Aspergillus fumigatus
<400> 53
   cgtcgacccc gccttt 16
<210> 54
   <211> 15
   <212> DNA
   <213> Aspergillus fumigatus
<400> 54
   caagggcctg tcacc 15
<210> 55
   <211> 21
   <212> DNA
   <213> Aspergillus fumigatus
<400> 55
   gagctgaaac tgccgttgat g 21
<210> 56
   <211> 20
   <212> DNA
   <213> Aspergillus fumigatus
<400> 56
   cctctgcggt actggtttcg 20
<210> 57
   <211> 17
   <212> DNA
   <213> Aspergillus fumigatus
<400> 57
   agcctgtgta catggat 17
<210> 58
   <211> 22
   <212> DNA
   <213> Aspergillus fumigatus
<400> 58
   cgttgatgca gcctgtgtac at 22
<210> 59
   <211> 15
   <212> DNA
   <213> Aspergillus fumigatus
<400> 59
   gacgcgcggc ttcct 15
<210> 60
   <211> 19
   <212> DNA
   <213> Aspergillus fumigatus
<400> 60
   ttctcgaaac cagtaccgc 19
<210> 61
   <211> 22
   <212> DNA
   <213> Aspergillus fumigatus
<400> 61
   gatgcagcct gtgtacatgg at 22
<210> 62
   <211> 15
   <212> DNA
   <213> Aspergillus fumigatus
<400> 62
   gacgcgcggc ttcct 15
<210> 63
   <211> 19
   <212> DNA
   <213> Aspergillus fumigatus
<400> 63
   tctcgaaacc agtaccgca 19
<210> 64
   <211> 19
   <212> DNA
   <213> Aspergillus fumigatus
<400> 64
   cctcgtatgg acgcggtaa 19
<210> 65
   <211> 23
   <212> DNA
   <213> Aspergillus fumigatus
<400> 65
   gactccacta tggcctgcta gtc 23
<210> 66
   <211> 14
   <212> DNA
   <213> Aspergillus fumigatus
<400> 66
   tgggccacat gttg 14
<210> 67
   <211> 22
   <212> DNA
   <213> Aspergillus fumigatus
<400> 67
   ccaatacgtc gcatacctgt ca 22
<210> 68
   <211> 19
   <212> DNA
   <213> Aspergillus fumigatus
<400> 68
   ggcgaccggc gtatacttc 19
<210> 69
   <211> 19
   <212> DNA
   <213> Aspergillus fumigatus
<400> 69
   agacatagac gactgccct 19
<210> 70
   <211> 17
   <212> DNA
   <213> Aspergillus fumigatus
<400> 70
   agcggcgcct tggttag 17
<210> 71
   <211> 18
   <212> DNA
   <213> Aspergillus fumigatus
<400> 71
   cgacgtcgca gccaaagt 18
<210> 72
   <211> 20
   <212> DNA
   <213> Aspergillus fumigatus
<400> 72
   ttcgctatgc atatagacct 20
<210> 73
   <211> 18
   <212> DNA
   <213> Aspergillus fumigatus
<400> 73
   gttcctcgag ccccgttt 18
<210> 74
   <211> 17
   <212> DNA
   <213> Aspergillus fumigatus
<400> 74
   tgtatggcgg gcattcg 17
<210> 75
   <211> 21
   <212> DNA
   <213> Aspergillus fumigatus
<400> 75
   caattgcaga aagtccctat a 21
<210> 76
   <211> 17
   <212> DNA
   <213> Aspergillus fumigatus
<400> 76
   tcgagccccg tttccaa 17
<210> 77
   <211> 17
   <212> DNA
   <213> Aspergillus fumigatus
<400> 77
   tgtatggcgg gcattcg 17
<210> 78
   <211> 19
   <212> DNA
   <213> Aspergillus fumigatus
<400> 78
   tgcagaaagt ccctatatg 19
<210> 79
   <211> 20
   <212> DNA
   <213> Aspergillus terreus
<400> 79
   ctcctcgcat ccagcgtaag 20
<210> 80
   <211> 22
   <212> DNA
   <213> Aspergillus terreus
<400> 80
   caggtcgaat tgggaagaag ac 22
<210> 81
   <211> 14
   <212> DNA
   <213> Aspergillus terreus
<400> 81
   ttgggcggcg ctac 14
<210> 82
   <211> 24
   <212> DNA
   <213> Aspergillus terreus
<400> 82
   tctgtgtatc acccagcttg attt 24
<210> 83
   <211> 20
   <212> DNA
   <213> Aspergillus terreus
<400> 83
   aacccgatca ggtccattga 20
<210> 84
   <211> 17
   <212> DNA
   <213> Aspergillus terreus
<400> 84
   cacggaaaca ttgtcgg 17
<210> 85
   <211> 22
   <212> DNA
   <213> Aspergillus terreus
<400> 85
   cgaatggata cgggaagaag ct 22
<210> 86
   <211> 19
   <212> DNA
   <213> Aspergillus terreus
<400> 86
   cgagcgaggc atcggtatg 19
<210> 87
   <211> 17
   <212> DNA
   <213> Aspergillus terreus
<400> 87
   ttgccattga caaactt 17
<210> 88
   <211> 21
   <212> DNA
   <213> Aspergillus terreus
<400> 88
   ggcaaaactc tgtcgcatac c 21
<210> 89
   <211> 21
   <212> DNA
   <213> Aspergillus terreus
<400> 89
   gcaccctcaa cagcagtgaa t 21
<210> 90
   <211> 15
   <212> DNA
   <213> Aspergillus terreus
<400> 90
   atgcctcgct cgcga 15
<210> 91
   <211> 21
   <212> DNA
   <213> Aspergillus terreus
<400> 91
   ctgctctatt gacccggtga a 21
<210> 92
   <211> 22
   <212> DNA
   <213> Aspergillus terreus
<400> 92
   tttttcccgc tcagagcata tc 22
<210> 93
   <211> 14
   <212> DNA
   <213> Aspergillus terreus
<400> 93
   accggctcgt ggtc 14
<210> 94
   <211> 19
   <212> DNA
   <213> Aspergillus terreus
<400> 94
   gcattgccgt gttcgatct 19
<210> 95
   <211> 22
   <212> DNA
   <213> Aspergillus terreus
<400> 95
   atccgccagt ttcgtagaaa ag 22
<210> 96
   <211> 14
   <212> DNA
   <213> Aspergillus terreus
<400> 96
   cgcaacaaag ggtg 14
<210> 97
   <211> 20
   <212> DNA
   <213> Aspergillus terreus
<400> 97
   aactggcgga tatcgctcat 20
<210> 98
   <211> 21
   <212> DNA
   <213> Aspergillus terreus
<400> 98
   agcatacccc tggaacacct t 21
<210> 99
   <211> 18
   <212> DNA
   <213> Aspergillus terreus
<400> 99
   cttcgctgga tacgctat 18
<210> 100
   <211> 20
   <212> DNA
   <213> Aspergillus terreus
<400> 100
   gcttctggtg gtgtggtcaa 20
<210> 101
   <211> 24
   <212> DNA
   <213> Aspergillus terreus
<400> 101
   gaccaccttc ccagtattca agtc 24
<210> 102
   <211> 14
   <212> DNA
   <213> Aspergillus terreus
<400> 102
   cgcaggtgac cgcc 14
<210> 103
   <211> 21
   <212> DNA
   <213> Aspergillus terreus
<400> 103
   ccgttttgag acagcgtatg g 21
<210> 104
   <211> 21
   <212> DNA
   <213> Aspergillus terreus
<400> 104
   acgtgggagt tgtcgtcact t 21
<210> 105
   <211> 15
   <212> DNA
   <213> Aspergillus terreus
<400> 105
   tacggagcga gcgct 15
<210> 106
   <211> 18
   <212> DNA
   <213> Aspergillus terreus
<400> 106
   ttcgcgtaac ggcacaag 18
<210> 107
   <211> 20
   <212> DNA
   <213> Aspergillus terreus
<400> 107
   ggcggtcaaa gcatcttttc 20
<210> 108
   <211> 14
   <212> DNA
   <213> Aspergillus terreus
<400> 108
   acccccggcg ttgt 14
<210> 109
   <211> 19
   <212> DNA
   <213> Aspergillus terreus
<400> 109
   cgcgtaacgg cacaagcta 19
<210> 110
   <211> 20
   <212> DNA
   <213> Aspergillus terreus
<400> 110
   ggcggtcaaa gcatcttttc 20
<210> 111
   <211> 14
   <212> DNA
   <213> Aspergillus terreus
<400> 111
   acccccggcg ttgt 14
<210> 112
   <211> 18
   <212> DNA
   <213> Aspergillus terreus
<400> 112
   tagacccccg gcgttgtt 18
<210> 113
   <211> 22
   <212> DNA
   <213> Aspergillus terreus
<400> 113
   cctactcgct ataggcggtc aa 22
<210> 114
   <211> 15
   <212> DNA
   <213> Aspergillus terreus
<400> 114
   cccactgaaa agatg 15
<210> 115
   <211> 24
   <212> DNA
   <213> Aspergillus terreus
<400> 115
   gatagaagaa tgccctctca gcat 24
<210> 116
   <211> 18
   <212> DNA
   <213> Aspergillus terreus
<400> 116
   cgccagtgga cgctcaac 18
<210> 117
   <211> 17
   <212> DNA
   <213> Aspergillus terreus
<400> 117
   cgacgaagac caccaca 17
<210> 118
   <211> 948
   <212> DNA
   <213> Aspergillus fumigatus
<400> 118
   atgactccgc tcgtcaaagt cggcagcctt ctggccgcat tgctctgggt cacctttggc        60
   atcgcgacag tcagcgccag catcccctca tccaaacgct tcgatctctc tgccccctcc       120
   gccgatctct accgtcacaa agccctccgc gatgacacag tccagcaagg attcgccttt       180
   gacaatgtca accgccgcct tttcgtcgcc cagcgccgcg acggctcctc cgaaacagcg       240
   ggcgacctaa caatcaccca gctcgacttt gacggcaact acgtcggaca catgtaccta       300
   aagagctttg gtcatggcgt ctccttcggc gcgcaggccg ttgggtccgc aacctacctc       360
   tggaccgagg tcgacgccaa cgccaacggc tacggcaagc aactcgcccg cttcaagttc       420
   gcctcgggca ccacgctgac cagctcctcc gctcaactgg caaagttcaa gcccgtcgcc       480
   gacgcaacgg agcatacctg cgccattgac ccggtgtaca accggctgat cgtccggtat       540
   cacctcagcg ggtcaaagca tatcgccgtg tatgatcttg ctgcggcgac gcgcggcgat       600
   ttctccgccc cgctggcgaa cttcaagcag ccggagttgg agacgctgtc gttgactttc       660
   cagggttatg cagcgtatgg acagtatctg tatctgctga cgggaacttc atatgcggcc       720
   agcggaggta aagtcaattc cgaggtaacc agtgttgaca tgaacacggg caaggtggtg       780
   caggggccca cgctcaccaa ggccggttcc acgctggagt ttagagagcc ggagggatta       840
   gcgatttaca agacggctgc ggggcaggtg aggttgttct tggggtttgc ttcggggaag       900
   gcgggtgatc gcaggtcgaa tctgttctac aagaatgtgt tggtttag                    948
<210> 119
   <211> 933
   <212> DNA
   <213> Aspergillus fumigatus
<400> 119
   atgcgagtct ccctctttgc tatcatcaca gcactaacat gtgtgattgc caaaatcccc      60
   gactctccac gattcgacct caccaaaccg tcgtatgacc tctggcggaa caagaaaacc     120
   gccgccaaaa ccgtccaaca gtcattcacc tttgacaacg tcaaccgccg cctgttcatc     180
   gtaaaccgtc gcgatggctc cgccctcgac tcaggagact tgacaatcag ccaactcgac     240
   ttctccggaa aggtcctcgg ctcaatgcac ctgctcggct gcggccacgg tgtgaacatc     300
   gccgcggaac caatcggaag cgatacgtat atctggagtg aaaccgacgc tgcagccagc     360
   ggatacggga cagcattgtg gaagttcaag ttccagaatg gcaagacgtt gaatagcgcc     420
   acggataagc gcaagcggat tgtgccgttg cccgagtttg acaggggcac tgccacaatt     480
   gaccccgttt ataagcggtt gatcgtgcga taccagacgg ggaccgtgca gaacatcgcc     540
   gcgtttgacc ttgctgaggc gagcgcaggc aatttttcga acccactggc caaatgggag     600
   attccgcatc tcgtcaagga gctcggggca aaggttaacg tctttcaggg atatacagct     660
   tacgggcggt atgtctattt cctgactggg gaatcttatg acgctagtgg tggtaagctg     720
   aattcagagg ttatggctct ggacttgaat actgggaagc ttgtgcaagg gcctgtcacc     780
   acaaaggcgg ggtcgacgtt gaaatttcgt gagccggagg ggatggcaat ctataagacg     840
   gccagtgggg aggtgaggct tttcttggga ttcgcgtcgg gggttgcagg agaccggagg     900
   agtaatctgt tttacaagaa cgcgctgata tag                                  933
<210> 120
   <211> 387
   <212> DNA
   <213> Aspergillus terreus
<400> 120
   atgcgtcctt tcctccaaac tattgtactc ctcgcatcca gcgtaagcct ggcttgggcg      60
   gcgctaccgt cttcttccca attcgacctg tcagcgccat cttacgacct cttccgcagc     120
   aagccccttg ctgacgcgac agtccaacaa ggcttctcct tcgacaatgt caatcatcgc     180
   ctctttgtcg cccaacgccg cgacgggtcc tccgaaaccg ccggcgatct gtgtatcacc     240
   cagcttgatt tccacggaaa cattgtcgga tcaatggacc tgatcgggtt tggccacggc     300
   gtgtcctttg ccgctcagcc agtcgtttgt caatggcaaa actctgtcgc ataccgatgc     360
   ctcgctcgcg aaattcactg ctgttga                                         387